# EUROPÄISCHE PATENTANMELDUNG

(11) **EP 0 884 030 A2**
(43) Veröffentlichungstag der Anmeldung: **16.12.1998**
(21) Anmeldenummer: 98108766.1
(22) Anmeldetag: 14.05.1998
(51) Int. Cl.: A61F 2/16

(54) **Intraoculare Linse**

(30) Priorität: 09.06.1997 DE 19724109
(71) Anmelder: Morcher GmbH, D-70374 Stuttgart (DE)
(72) Erfinder: Teichmann, Klaus D., 11462 Riyadh (SA)
(74) Vertreter: Patentanwalts-Partnerschaft Rotermund + Pfusch

(57) **Zusammenfassung**

Am Linsenrand sind Haptikelemente (2) nach Art von C-Federbügeln mit linsenseitigem Krümmungszentrum angeordnet. Diese Haptikelemente besitzen in Umfangsrichtung der Linse voneinander beabstandete, zur Linsenebene zumindest annähernd parallele Bohrungen (3) zur Durchführung einer Fadenschlinge für eine vorzugsweise transsklerale Nahtfixation im Auge.

## Beschreibung

Die Erfindung betrifft eine intraoculare Linse, geeignet als Hinterkammerlinse zur Fixation im Sulcus ciliaris bzw. im eröffneten Kapselsack oder auf der Pars plana, mit vom Linsenrand nach radial auswärts abstehenden Haptikelementen, welche nach Art eines C-Federbügels mit linsenseitigem Krümmungszentrum ausgebildet sind.

Derartige Linsen sind in vielfältigen Ausführungsformen bekannt, vgl. beispielsweise DE 36 27 488 A1, DE 39 01 435 A1, US 4,409,690, US 5,336,262, US 5,480,426 und den Aufsatz 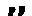 Pars Plana Fixation of Posterior Chamber Intraocular Lenses" von K. D. Teichmann in OPHTHALMIC SURGERY (1994) 549 bis 553.

Grundsätzlich können intraoculare Linsen in verschiedener Weise angeordnet werden.

Neben Linsen zur Fixation im Sulcus ciliaris bzw. im Kapselsack sind auch im Kammerwinkel der Vorderkammer des Auges abgestützte oder an der Iris fixierte Intraocularlinsen bekannt.

Im Hinblick auf potentielle Störungen der Blut-Kammerwasser-Schranke, deren Aufrechterhaltung ganz wesentlich über die Langzeitverträglichkeit einer intraocularen Linse entscheiden dürfte, gilt die Fixation im Kapselsack als optimale Lösung.

Wenn allerdings hinreichende Kapselsackstrukturen fehlen, weil der Kapselsack weitestgehend oder vollständig entfernt werden mußte, wird in der Regel eine Sulcusfixation der Linse vorgenommen. Statt dessen kann auch eine - bislang allerdings wenig gebräuchliche - Fixation auf der Pars plana erfolgen.

Unter ungünstigen Operationsbedingungen, insbesondere bei weitgehend fehlenden Kapselsackstrukturen, ist eine Nahtfixation der implantierten Intraocularlinse wünschenswert, d.h. eine transsklerale Fixation im Sulcus ciliaris oder auf der Pars plana.

Bei den Haptikelementen der Linsen, die in den oben genannten Druckschriften dargestellt werden, muß der Faden bei einer Nahtfixation zumindest bereichsweise senkrecht zur Äquatorebene der Linse geführt werden. Dies kann operationstechnische Schwierigkeiten mit sich bringen. Insbesondere läßt es sich des öfteren nicht vermeiden, daß durch die Nahtfixation Kippmomente auf die Linse bzw. deren Haptikelemente einwirken.

Aufgabe der Erfindung ist es nun, intraokulare Linsen mit einer für Nahtfixationen optimalen Haptik zu versehen.

Dies wird bei der Erfindung dadurch erreicht, daß zumindest ein Haptikelement an einem - zumindest nach Implantation der Linse im Auge - zur Linsenachse konzentrischen Bereich zwei zur Durchführung einer Fadenschlinge geeignete, zur Befestigungszone bzw. -fläche im Auge zumindest annähernd senkrechte und zur Äquatorebene der Linse etwa parallele Bohrungen aufweist, die voneinander in Umfangsrichtung der Linse beabstandet sind.

Die Erfindung beruht auf dem allgemeinen Gedanken, die Haptikelemente derart auszubilden, daß eine Nahtfixation mittels einer Fadenschlinge ermöglicht wird, deren Schlingenebene etwa parallel zur Linsenebene oder derartig zur Linsenebene geneigt ist, daß sich die Linsenebene und die Schlingenebene an einer Linie durchsetzen, die tangential zu einem zum Linsenzentrum konzentrischen Kreis verläuft. Eine solche Fadenschlinge kann auf die Haptik praktisch keinerlei Kräfte ausüben, die die Linse zu verkippen suchen. Gleichzeitig ist eine besonders sichere Fixation gewährleistet, da die Fadenschlinge zwei in Umfangsrichtung der Intraocularlinse voneinander beabstandete Orte an der Sklera des Auges nahe der peripheren Iris durchsetzen kann. Dabei ist besonders vorteilhaft, daß der Knoten der Fadenschlinge durch Rotation der Schlinge im Skleragewebe versenkt werden kann, um ein späteres Durchscheuern und Durchstechen der Fadenenden zu verhindern.

Gemäß einer bevorzugten Ausführungsform der Erfindung kann vorgesehen sein, die Bohrungen in Draufsicht auf die Linse unter einem spitzen Winkel zwischen 0° und 60° - z.B. etwa 45° - anzuordnen, derart, daß der Scheitelpunkt des Winkels auf der Konkavseite des die Bohrungen aufweisenden Bereiches des jeweiligen C-Federbügels liegt. Dadurch kann einer mechanischen Beschädigung des Fixationsfadens sowohl bei der Rotation der Fadenschlinge als auch später vorgebeugt werden.

Außerdem kann es vorteilhaft sein, wenn der die Bohrungen aufweisende Bereich des C-Federbügels flachbandartig mit zur Linsenebene etwa senkrechter bzw. zur Fixationsfläche im Auge etwa paralleler Bandebene ausgebildet ist. Dabei kann durch entsprechend große Bemessung der Breite des Flachbandes eine besonders geringe Flächenpressung zwischen dem jeweiligen Haptikelement und dem zur Nahtfixierung dienenden Gewebeteilen des Auges gewährleistet werden, wie es zur Vermeidung von postoperativen Komplikationen wünschenswert ist.

Im übrigen wird hinsichtlich bevorzugter Merkmale der Erfindung auf die Ansprüche sowie die nachfolgende Erläuterung einer besonders bevorzugten Ausführungsform verwiesen, die anhand der Zeichnung beschrieben wird.

Dabei zeigt
- Fig. 1: eine Draufsicht auf die erfindungsgemäße Linse,
- Fig. 2: eine Seitenansicht dieser Linse,
- Fig. 3: einen Längsschnitt der hornhautseitigen Vorderhälfte eines Auges mit im Sulcus ciliaris fixierter Intraocularlinse und
- Fig. 4: eine der Fig. 3 entsprechende Darstellung bei Fixation der Intraocularlinse auf der Pars plana.

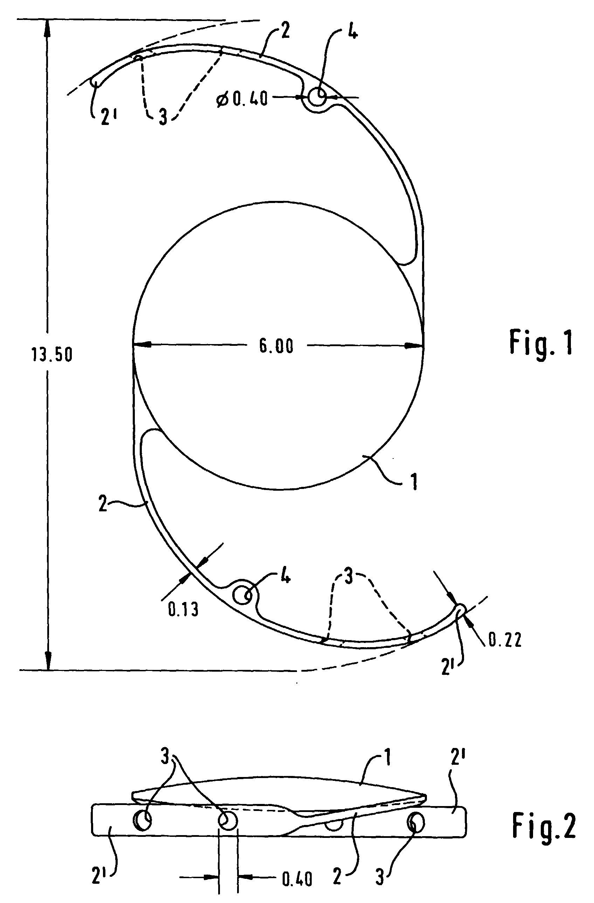

Die in den Fig. 1 und 2 dargestellte Intraocularlinse 1 ist als bikonvexe Linse ausgebildet. Jedoch sind andere Linsenformen möglich bzw. je nach Erfordernissen notwendig. Beispielsweise könnte die Linse auch eine konkave und eine konvexe Seite aufweisen.

Zur Halterung der Linse 1 im Auge sind am Linsenrand Haptikelemente 2 angeordnet bzw. angeformt, welche als C-förmige Bügel ausgebildet sind, die ein freies Ende und ein tangential in den Linsenrand übergehendes festes Ende aufweisen. Das Krümmungszentrum der Bügel 2 liegt jeweils linsenseitig.

Im übrigen können die Bügel 2 gegenüber der Äquatorebene der Linse 1 um einen kleinen Winkel von bis zu etwa 10° nach vorne (für die Sulcusfixation) oder nach hinten (für die Fixation auf der Pars plans) abgewinkelt sein, jedoch derart, daß die Längsachsen der Endbereiche 2' der Haptikelemente 2 in einer zur Äquatorebene der Linse 1 parallelen Ebene erstreckt sind. Dies ist gleichbedeutend damit, daß die zwischen den Endbereichen 2' und dem Rand der Intraocularlinse 1 verbleibenden Teile der Haptikelemente 2 sowohl gegenüber der Äquatorebene der Linse 1 als auch gegenüber der von den Endbereichen 2' aufgespannten Ebene abgewinkelt sind, wobei die zwischen den Endbereichen 2' und der Linse 1 erstreckten Teile der Haptikelemente 2 in einer der Umfangsfläche eines flachen Kegelstumpfes entsprechenden bzw. angenäherten Ebene erstreckt sind.

Die an den Linsenumfang anschließenden Teile der Haptikelemente 2 besitzen einen im wesentlichen kreisförmigen Querschnitt mit sehr geringem Durchmesser von beispielsweise 0,13 mm bis 0,20 mm, während die Endbereiche 2' flachbandartig ausgebildet sind, und zwar mit einer zur Äquatorebene der Linse 1 etwa senkrechten bzw. zur Befestigungszone im Auge etwa parallelen Bandebene. Die Breite der Endstücke 2' quer zur Äquatorebene der Linse 1 ist deutlich größer als der Durchmesser der vorgenannten Teile der Haptikelemente 2 und kann beispielsweise zwischen 0,6 und 0,7 mm liegen. Die freien Enden der flachbandartigen Endbereiche 2' sind gemäß Fig. 1 etwas verdickt und gut gerundet.

In den Endbereichen 2' sind jeweils zwei zur Äquatorebene der Linse 1 etwa parallele bzw. zur Linsenachse radiale Bohrungen 3 angeordnet, deren Achsen einen spitzen Winkel zwischen 0° und 60°, z.B. einen Winkel von etwa 45°, zueinander bilden können, dessen Scheitelpunkt linsenseitig der Endbereiche 2' liegt.

Auf der dem Linsenrand zugewandten Seite der Haptikelemente 2 können entweder an deren freien Enden oder - vorzugsweise - etwa mittig zwischen dem freien und dem festen Ende des jeweiligen Haptikelementes 2 und damit vorzugsweise außerhalb des jeweiligen flachbandartigen Endbereiches 2' kleine Ösen 4 mit zur Ebene des jeweiligen Haptikelementes 2 etwa senkrechter Öffnungsachse angeformt sein.

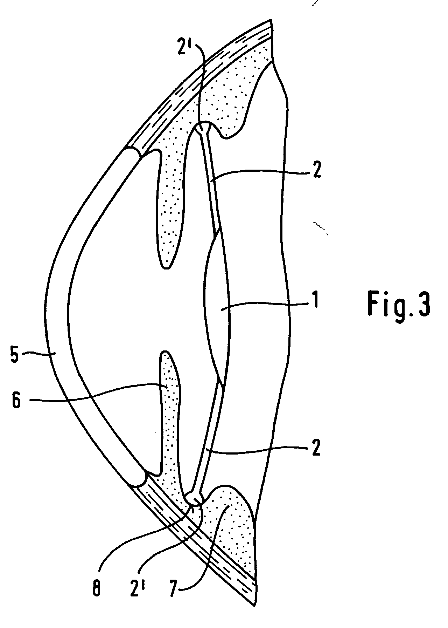

Die in Fig. 3 dargestellte, in Längsrichtung geschnittene Vorderhälfte eines Auges zeigt dessen Hornhaut 5, dessen Iris 6 sowie Teile seiner Ciliarmuskulatur 7, welche normalerweise den Kapselsack der natürlichen Linse des Auges am Linsenäquator haltert.

Im vorliegenden Falle wird davon ausgegangen, daß die natürliche Linse sowie der Kapselsack operativ entfernt werden mußten.

Wenn in einem solchen Fall eine künstliche Intraocularlinse der in den Fig. 1 und 2 dargestellten Art implantiert werden soll, können die Haptikelemente 2 der Intraocularlinse 1 im Sulcus ciliaris 8 zwischen der Iris 6 und der Ciliarmuskulatur 7 mittels einer transskleralen Naht fixiert werden. Dazu kann bei der erfindungsgemäßen Linse eine die Bohrungen 3 der Endbereiche 2' der Haptikelemente 2 (vgl. Fig. 1) sowie die innere Schicht der Sklera durchsetzende Fadenschlinge vorgesehen sein, wobei der Knoten der Fadenschlinge durch Rotation der Schlinge im Skleragewebe versenkt werden kann, ohne daß ein Skleralappen zur Überdeckung des Knotens erstellt werden müßte.

Da die von der Fadenschlinge durchsetzten Bohrungen 3 (vgl. Fig. 1) an den Endbereichen 2' der Haptikelemente 2 in Umfangsrichtung der Linse 1 voneinander beabstandet sind, können durch die Fadenschlingen praktisch keinerlei Torsionskräfte auf die Haptikelemente 2 ausgeübt werden. Dementsprechend kann die Intraocularlinse 1 vergleichsweise stabil und unverkippbar in der jeweils gewünschten Position fixiert werden.

Aus Fig. 3 ist des weiteren ersichtlich, daß die Intraocularlinse 1 aufgrund der Schrägstellung der Haptikelemente 2 gegenüber der Äquatorebene der Linse 1 einen relativ großen axialen Abstand von der Iris 6 aufweisen kann. Dies ist vorteilhaft, weil damit Irritationen der Iris 6 durch die Intraocularlinse 1 sicher vermieden werden können.

Da der Durchmesser des Sulcus ciliaris 8 geringer ist als der Abstand der freien Enden der Haptikelemente 2 in Durchmesserrichtung der Linse 1 in Fig. 1, werden die Haptikelemente 2 bei der Implantation federnd radial einwärts gedrängt, so daß die die Bohrungen 3 aufweisenden Endbereiche 2' mit einem gewissen Kraftschluß in den Sulcus ciliaris 8 gedrängt werden.

Die Ösen 4 an den Haptikelementen 2 erleichtern die Manipulation der Intraocularlinse 1 bei der Implantation und können beispielsweise zum Eingriff von Operationswerkzeugen dienen.

Die in den Fig. 1 und 2 eingetragenen Bemessungen, insbesondere der Haptikelemente 2, haben sich bei der Fixation der Intraocularlinse 1 im Sulcus ciliaris als vorteilhaft erwiesen, zumindest dann, wenn die Intraocularlinse 1 sowie die Haptikelemente 2 aus PMMA (Polymethylmethacrylat) oder einem sonstigen Acrylglas hergestellt sind.

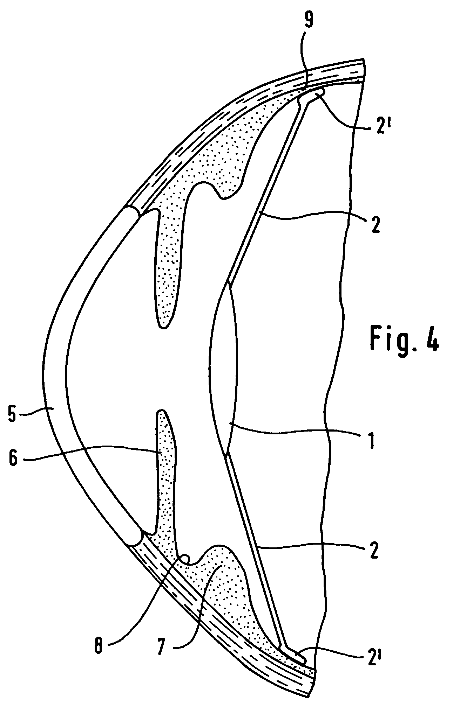

Andere Bemessungen sind jedoch grundsätzlich möglich und für eine Fixation der Intraocularlinse 1 auf der Pars plans 9 - vgl. Fig. 4 - zweckmäßig sowie vorteilhaft, wie weiter unten ausgeführt wird.

Da die Pars plana 9 relativ eben bzw. glatt und frei von gefäßreichen Muskelschichten ist, eignet sie sich für das Durchstechen des Fixationsfadens sowie das Anlagern der Endbereiche 2' der Haptikelemente 2 besonders gut.

In Anpassung an den relativ großen Durchmesser der Pars plana 9 sollte der Durchmesser der Intraocularlinse 1 unter Einschluß der Haptikelemente entsprechend groß sein und z.B. bei etwa 16 mm bis 17 mm liegen. Die Haptikelemente 2 sind gegenüber der Linsenebene bevorzugt nach rückwärts abgewinkelt, so daß die Intraocularlinse 1 dem Platz der (entfernten) natürlichen Linse zumindest angenähert ist. Die Endstücke 2' der Haptikelemente 2 sind relativ zur Linsen-ebene geneigt angeordnet, so daß die Bandebene der Endstücke 2' etwa parallel zur Pars plana 9 erstreckt ist. Im übrigen sind ähnliche Bemessungen, wie sie in Fig. 1 angegeben sind, zweckmäßig und vorteilhaft.

## Patentansprüche

1. Intraoculare Linse, geeignet als Hinterkammerlinse zur Fixation im Sulcus ciliaris bzw. im eröffneten Kapselsack oder auf der Pars plana, mit vom Linsenrand nach radial auswärts abstehenden Haptikelementen, welche nach Art eines C-Federbügels mit linsenseitigem Krümmungszentrum ausgebildet sind,
**wobei,**
zumindest ein Haptikelement (2) an einem - zumindest nach Implantation der Linse (1) im Auge - zur Linsenachse konzentrischen Bereich (2') zwei zur Durchführung einer Fadenschlinge geeignete, zur Befestigungszone bzw. -fläche im Auge zumindest annähernd senkrechte und zur Äquatorebene der Linse (1) etwa parallele Bohrungen (3) aufweist, die voneinander in Umfangsrichtung der Linse (1) beabstandet sind.

2. Linse nach Anspruch 1,
**dadurch gekennzeichnet,**
daß die Bohrungen (3) in Draufsicht auf die Linse (1) unter einem spitzen Winkel angeordnet sind, dessen Scheitelpunkt auf der linsenseitigen Konkavseite des die Bohrungen (3) aufweisenden Bereiches (2') des jeweiligen C-Federbügels (2) liegt.

3. Linse nach Anspruch 2,
**dadurch gekennzeichnet,**
daß der Winkel zwischen 0° und 60° liegt bzw. ein Maß von etwa 45° besitzt.

4. Linse nach einem der Ansprüche 1 bis 3,
**dadurch gekennzeichnet,**
daß der die Bohrungen (3) aufweisende Bereich (2') flachbandartig mit zur Befestigungszone bzw. -fläche im Auge etwa paralleler und zur Äquatorebene der Linse (1) etwa senkrechter Bandebene ausgebildet ist.

5. Linse nach einem der Ansprüche 1 bis 4,
**dadurch gekennzeichnet,**
daß am C-Federbügel (2) eine vom Bereich zwischen den Bohrungen (3) beabstandete Öse (4) mit zur Linsenebene bzw. zur Ebene der Haptikelemente (2) etwa senkrechter Achse angeordnet ist.

6. Linse nach einem der Ansprüche 1 bis 5,
**dadurch gekennzeichnet,**
daß ein C-Federbügel bzw. alle C-Federbügel (2) jeweils ein am Linsenumfang gehaltertes sowie ein freies Ende aufweisen.

7. Linse nach den Ansprüchen 5 und 6,
**dadurch gekennzeichnet,**
daß die Öse (4) jeweils an einem Mittelbereich des C-Federbügels zwischen dem freien und dem festen Ende angeordnet ist.

8. Linse nach einem der Ansprüche 1 bis 7,
**dadurch gekennzeichnet,**
daß die Längsachsen der die Bohrungen (3) aufweisenden Bereiche (2') der Haptikelemente (2) in einer zur Linsenebene etwa parallelen Ebene und die diese Bereiche (2') mit der Linse (1) verbindenden Teile der Haptikelemente (2) gegenüber der Linsenebene zur einen Seite der Linse (1) abgewinkelt angeordnet sind.
